# EUROPEAN PATENT APPLICATION

(11) **EP 4 789 639 A1**
(43) Date of publication of application: **12.08.2026**
(21) Application number: 24386151.5
(22) Date of filing: 20.12.2024
(51) Int. Cl.: A61F 2/16

(54) **SUTURELESS SCLERAL FIXATED IOL WITH HAPTICS DESIGNED FOR SUBCONJUNCTIVAL PLACEMENT**

(71) Applicant: National and Kapodistrian University of Athens, 10679 Athens (GR)
(72) Inventor: MPARLAMPA, Aikaterini, 11527 Athens (GR); DOUMAZOS, Leonidas, 11527 Athens (GR); KARAGEORGIOU, Georgia, 11527 Athens (GR); PETROU, Petros, 11527 Athens (GR); DOUMAZOS, Spyros, 11527 Athens (GR); KANDARAKIS, Stylianos, 11527 Athens (GR); NTIKOS, Stergios, 11527 Athens (GR)
(74) Representative: Minas, Nikolaos

(57) **Abstract**

The present invention provides an intraocular lens (IOL) (10) specifically designed for posterior chamber sutureless scleral fixation. A pinhead-shaped haptic design (12) eliminates the need for scleral flaps, thereby simplifying the surgical procedure and reducing operative time while enhancing postoperative outcomes.

## Description

### Field

The present invention relates to a sutureless scleral fixated intraocular lens (IOL) with haptics designed for subconjunctival placement. The IOL of the present invention comprises haptics having a pinhead-shaped anchoring element for anchoring designed for subconjunctival fixation without creation of scleral flaps.

### Background

Cataract surgery is one of the most frequent ophthalmic procedures worldwide. It is usually indicated in elderly patients in order to address poor vision due to cataract formation. The solution is basically removing the cataract and inserting an intraocular lens (IOL) which is fixated in the capsular bag; i.e.. a routine cataract surgery case. Problems arise when intraocular complications occur due to capsular insufficiency. Zonular dehiscence of more than 6-7 clock hours disables insertion of a posterior chamber IOL in the bag or in the sulcus. Other late situations where alternative methods of IOL fixation should be deployed are in cases of traumatic aphakia due to lens or IOL dislocation. Therefore, alternative methods of IOL fixation need to be devised. Up until recently Anterior chamber (AC) IOLs were used fixating their haptics at the iridocorneal angle. Alternatively, iris fixated IOLs have been used either anterior or posterior to the iris. These solutions have addressed the problem of IOL fixation, however, cause various postoperative complications. To this end other methods of IOL fixation have been tried and tested like placing scleral sutures for scleral fixation of an IOL or Sutureless as the Yamane technique forming button tips at the end of a three-piece IOL. EP 4338706 A1 ,discloses a solution for sutureless scleral fixated intraocular lens (IOL) using T-shaped haptics designed for posterior chamber implantation. While this design eliminates sutures, it requires the creation of scleral flaps to cover the haptics after they are externalized through sclerotomies, adding significant surgical complexity. Moreover, improper flap coverage can lead to postoperative risks such as hypotony, haptic exposure, and IOL tilt.

Therefore, there is a need for an improved IOL design that is simple to insert, and drastically decrease the surgical time required and associated complications.

### Summary

The present invention provides an intraocular lens (IOL) designed for posterior chamber sutureless scleral fixation. This invention introduces a novel pinhead-shaped haptic design that eliminates the need for scleral flaps, thereby simplifying the surgical procedure and reducing operative time while enhancing postoperative outcomes.

According to an embodiments of the present invention, the intraocular lens (IOL) comprises an optic plate and two haptics extending from opposite sides of the optic plate. Each haptic terminates in a pinhead-shaped anchoring element having a round terminal portion configured to rest flat against the sclera. The pinhead-shaped anchoring elements, which also may be referred to as plugs in the literature, are designed to cover the sclerotomy, ensuring stable anchoring, minimizing the risk of postoperative hypotony, and preventing exposure under the conjunctiva

According to an embodiment of the present invention, the haptics and/or the entire IOL are made from from a biocompatible, flexible, and foldable material, providing flexibility and ease of insertion. The material may be a hydrophilic or a hydrophobic acrylic material.

According to an embodiment of the present invention, the pinhead-shaped anchoring elements are designed to fit through sclerotomies created using 23G or 25G trocars, resting flush under the conjunctiva without inducing bulging. The curved surface of the anchoring elements minimizes friction during insertion and manipulation, while its structural design ensures resistance to mechanical stress and prevents breakage during surgical handling.

According to an embodiment of the present invention, the optic plate of the IOL is available in diameters ranging from 6 mm to 8 mm and can be configured for monofocal, toric, extended depth of focus (EDOF), or combined EDOF-toric optics to address a wide range of refractive needs. The haptics are anteriorly angled relative to the optic plate to ensure anatomical alignment and optimal centration within the posterior chamber.

The present invention is also suited for cases requiring secondary IOL implantation, such as aphakia, lens subluxation, traumatic cataracts, or conditions with advanced zonular dehiscence. By eliminating the need for scleral flaps and sutures, this IOL design simplifies the implantation process, reduces complications, and provides improved outcomes for patients with insufficient capsular support.

### Brief Description of the drawings

The following drawings are provided as an example to explain further and describe various aspects of the invention.
Figure 1 shows a perspective view of an intraocular lens (IOL) according to embodiments of the present invention
Figures 2A to 2D show different views of the haptic anchoring element according to embodiments of the present invention,

### Detailed Description

For the purposes of promoting an understanding of the principles of the present disclosure, reference will now be made to the implementations illustrated in the drawings, and specific language will be used to describe the same. It will nevertheless be understood that no limitation of the scope of the disclosure is intended. Any alterations and further modifications to the described devices, methods and any further application of the principles of the present disclosure are fully contemplated as would normally occur to one skilled in the art to which the disclosure relates. In particular, it is fully contemplated that the features, components, and/or steps described with respect to one implementation may be combined with the features, components and/or steps described with respect to other implementations of the present disclosure.

In the detailed description and figures of this disclosure, reference numerals are used to denote corresponding elements that are similar in structure and function across multiple embodiments. It should be understood that when a reference numeral appears in different figures or embodiments, it refers to the same or substantially similar element unless otherwise specified. This convention is used to simplify the explanation and enhance the clarity of the various embodiments presented.

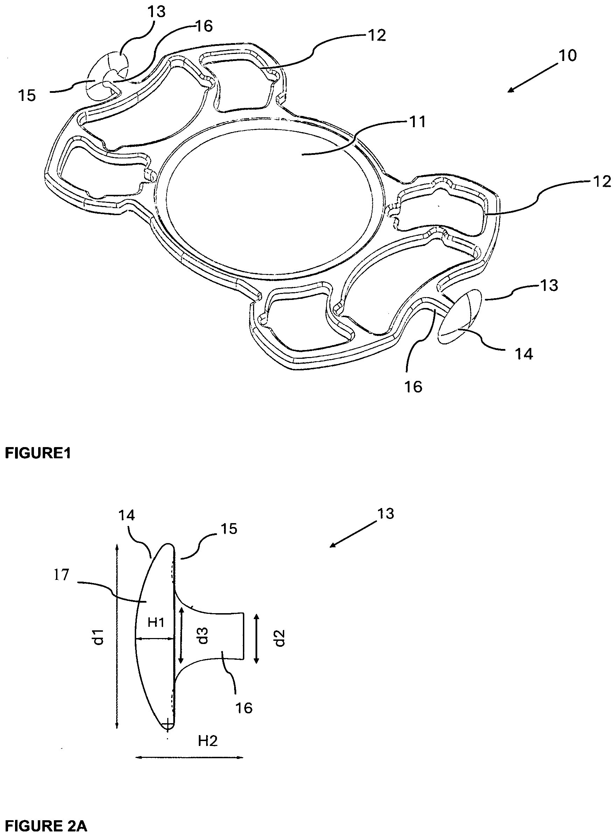

The present invention will be described herein with reference to figures 1 and 2A to 2D. The proposed IOL 10 of the present invention provides an improved haptics design for subconjunctival fixation that eliminates the creation of scleral flaps. The improved haptics design greatly simplifies the surgical procedure, improve the recovery time of the patient, and minimising post-surgical complications.

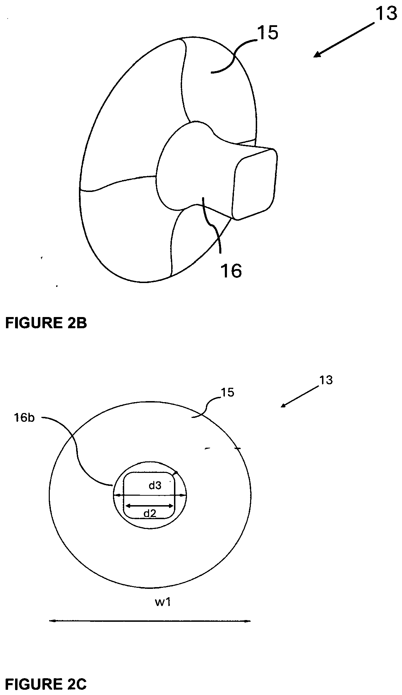

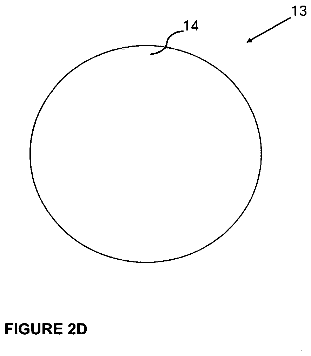

As shown in Figure 2, The IOL 10 is provided with an optic plate 11 and two haptics 12. The proposed IOL haptics comprise pinhead-shaped anchoring element 13. Each anchoring element is provided with a rounded terminal portion 17 and a stem 16. According to embodiments of the present invention, the terminal portion 17 of the anchoring element 13 has a diameter (d1) preferably between 1.20mm and 1.70mm, and more preferably between 1.50mm and 1.60mm. The terminal portion 17 is provided with an upper surface 14 and a bottom surface 15. The bottom surface 15 may be substantially flat and configured to cover the sclerotomy after insertion of the IOL 10. As shown in figure 2A, the upper surface 14 of the terminal portion 17 may have a convex profile, although other suitable profiles may be used such as concave, bowl-shaped, and the like. According to embodiments of the present invention, the terminal portion 17 may have a height (h1), defined between the upper surface 14 and the bottom surface 15, in the range of 0.20mm to 0.30mm.

According to embodiments of the present invention, the anchoring elements 13 may be provided with a terminal portion 17 having a diameter of 1.58mm. In comparison, the T-Shaped haptics of prior art solutions, such as the one present in EP 4338706, are longer at 2mm length. To put things into perspective, a 23G sclerotomy will create a 0.64mm wide wound in the sclera. As the haptic will assume its position through the sclerotomy and lie on the sclera, the pinhead-shaped anchoring elements 13 of the present invention are designed to cover the sclerotomy, covering the wound completely - 360 degrees - as well as supporting the IOL 10 in position. The conjunctiva will lie on top of the haptic 12, following the curvature of the sclera with no bulging and a smooth final surface. On the contrary, the T-shaped haptics of the prior art solutions require the formation of scleral flaps to avoid exposure and hold the IOL in position. Therefore, the proposed pinhead-shaped haptic design provides the advantages of modifying the surgical approach required for the IOL placement making it easier, faster and more efficient.

As shown in figures 1 and 2A-2C, the anchoring elements 13 comprise a stem 16, extending between the rounded terminal portion 17 and the haptic extremity. The stem 16 is designed to improve stability and strength of the haptic 12, so as to minimise haptic breakage during insertion of the IOL 10. According to embodiments of the present invention, the stem 16 may be provided with a flared design, tapering outwardly at it approaches the terminal portion, as shown in figures 2A-2C. The stem 16 is designed such that its cross-sectional dimensions increase progressively along its length towards the terminal portion 17. The cross-sectional profile of the stem 16 may also progressively change from a rectangular profile with rounded edges at the location of the haptic extremities to a substantial rounded profile at the location of the terminal portion 17. As shown in figures 2A and 2B, the diameter of the stem closer to the terminal portion, denoted by d3, is larger than the diameter of the stem closer to the haptic extremity, denoted by d2. For example, diameter d2 may be between 0.30mm to 0.40mm, while the diameter d3 may be between 0.50mm to 0.60mm. The skilled person in the art may adapt the dimensions of the stem in order to increase the strength and stability of the haptics while maintaining their functionality. According to embodiments of the present invention, the height of the anchoring element, denoted by H2 in Figure 2A, may be between 0.80mm and 0.90mm. Overall, the diameter of the stem 16 is smaller compared of the diameter of the terminal portion 17. For example, the diameter of the stem 16 may be 30-70% smaller than diameter of the terminal portion. In the case of a stem 16 with a flared design, the diameter of the stem 16 is not contact along its length.

According to embodiments of the present invention, the material of the haptics may be hydrophilic allowing for a smooth, curved, frictionless haptic surface. These changes will allow the haptic to lie flat onto the sclera minimizing friction and will be the first scleral fixated IOL designed specifically for subconjunctival placement. In other embodiments, the haptics material may be hydrophobic.

For the surgical procedure for the proposed IOL design, the sclerotomies used for the externalization of the rounded pinhead shaped haptics will be the same as the sclerotomies formed in the placement of the trocars. Three trocars (23G or 25G) are inserted 3mm from the limbus, displacing the conjunctiva and are placed in such a way in order for two of the trocars to be directly opposite to each other. Vitrectomy is performed as usual and when it is time to insert the IOL 10 the infusion line is switched to the upper temporal trocar in order to free up the two opposite trocars. The IOL is injected through a corneal incision of approximately 2.75mm and using an intraocular forceps through the inferior trocar the leading haptic 12 is grasped and externalized together with the trocar removal. The pinhead shaped anchoring element 13 of the haptic 12 will rest flat onto the sclera covering the sclerotomy opening. The haptic will be covered with conjunctiva. The trailing haptic 12 is externalized in a similar fashion through the remaining superior nasal trocar. At the end of surgery, an amount of a dispersive viscoelastic material is injected into the anterior chamber. For example, 1 ml of viscoelastic material is injected in the anterior chamber.

Since mostly Vitreoretinal surgeons (VR) surgeons are requested to deal with complicated cases of phacodonesis or IOL dislocations, performing a complete vitrectomy as part of the insertion of a scleral fixated IOL is deemed preferable. In this way, the retinal periphery is intraoperatively examined for tears, but prompt treatment can be performed and greatly reduce the risk of postoperative retinal detachment. Therefore, since sclerotomies are formed to insert the trocars, these sclerotomies can be used to fixate the haptics of the new proposed IOL, sub-conjunctively. Placing the two trocars opposite to each other can allow for proper IOL fixation. Externalization of the haptic occurs using an intraocular forceps through the trocar, which is removed as the trocar is expressed on the surface of the sclera. Overall, the proposed procedure for inserting the IOL of the present invention without the opening of the conjunctiva, greatly decreases surgical time which consequently reduces intraoperative and postoperative complications. The proposed haptic design does not require unnecessary openings or handling of the conjunctiva and sclera, compared to the prior art solutions.

Overall, this new IOL design has potentially several advantages.
a. First and foremost, it addresses the refractive error of the patient lacking an IOL. Apart from monofocal optics It can also come in a toric variant, EDOF or combined EDOF-toric scleral fixated IOL. Therefore, this IOL can accommodate even demanding patients requiring premium IOL insertion even though lack of capsular support hindered their request.
b. Secondly, because of sutureless scleral fixation the IOL stability and centration is perfect, eliminating postoperative IOL tilt and suture related complications.
c. Thirdly, specifically because of the new haptic design the sclerotomies are covered reducing potential postoperative hypotony, while allowing for subconjunctival placement without increasing risk of exposure. Additionally, they are potentially more resistant to haptic breaks during placement.
d. Finally, surgical time can be greatly reduced as well as intraoperative technical difficulties can be eliminated letting room for a simpler technique resulting into fewer risks of complications and a better final outcome for the patient. Scleral openings are kept to a minimum while no sutures are required in the procedure.

## Claims

1. An intraocular lens (IOL) (10) for posterior chamber sutureless scleral fixation, comprising:
an optic plate (11) ; and
two haptics (12) extending from opposite sides of the optic plate (11) , each haptic (12) comprising at its extremity a pinhead-shaped anchoring element (13) for anchoring to the sclera;
wherein the pinhead-shaped anchoring element (13) comprises a rounded terminal portion (17) having an upper surface (14) and a bottom surface (15) , wherein the bottom surface (15) is configured, when the IOL (10) is fixed in the posterior chamber, to cover the sclerotomy and rest flat on the sclera so as to eliminate the need for scleral flaps.

2. The IOL (10) of clam 1, wherein the upper surface (14) of each anchoring element (13) has a curved profile.

3. The IOL (10) of claim 2, wherein the upper surface (14) of each anchoring element (13) has a convex shaped profile.

4. The IOL (10) of claim 2, wherein the upper surface (14) of each anchoring element (13) has a concave or bowl shaped profile.

5. The IOL (10) of any one of the preceding claims wherein the pinhead-shaped anchoring elements (13) are designed to fit through sclerotomies created using 23G or 25G trocars, resting flush under the conjunctiva without inducing bulging.

6. The IOL (10) of any one of the preceding claims, wherein the haptics (120 are made from a flexible and foldable material.

7. The IOL(10) of claim 6, wherein the material is hydrophilic or hydrophobic.

8. The IOL (10) of any one of the preceding claims, wherein the pinhead-shaped anchoring elements (13) comprise a stem (16) extending between the haptic extremities and the terminal portion (17) .

9. The IOL (10) of claim 8, wherein the cross-sectional dimensions of the stem (16) is smaller than the diameter of the terminal portion (17).

10. The IOL (10) of claim 9, wherein the cross-sectional diameter of the stem (16) is between 30.0-70.0% smaller compared to the diameter of the terminal portion (17) .

11. the IOL (10) of any one of claims 8 to 10, wherein the stem (16) has a flared design with its cross-sectional diameter increasing progressively along its length towards the terminal portion (17).

12. The IOL (10) of claim 11, wherein the stem (16) has a substantially rectangular profile at the location of the haptic extremities, and a substantial rounded profile at the location of the terminal portion.

13. The IOL (10) of claim of claim 12, wherein the rectangular profile comprises rounded edges.

14. The IOL (10) of any one of the preceding claims, wherein the terminal portion of the pinhead-shaped anchoring element (13) is configured to provide resistance against rotational displacement within the sclera.

15. The IOL (10) of any one of the preceding claims, wherein each haptic (12) is angled anteriorly relative to the optic plate by 10° to 15°, ensuring anatomical alignment and optimal centration of the IOL in the posterior chamber.
